(19) Europäisches Patentamt
European Patent Office
Office européen des brevets

(11) EP 1 261 861 B1

(12) **EUROPÄISCHE PATENTSCHRIFT**

(45) Veröffentlichungstag und Bekanntmachung des Hinweises auf die Patenterteilung:
**29.09.2004 Patentblatt 2004/40**

(21) Anmeldenummer: **01905800.7**

(22) Anmeldetag: **19.02.2001**

(51) Int Cl.[7]: **G01N 27/327**, G01N 33/86, C12Q 1/00

(86) Internationale Anmeldenummer:
**PCT/EP2001/001848**

(87) Internationale Veröffentlichungsnummer:
**WO 2001/063271 (30.08.2001 Gazette 2001/35)**

(54) **ELEKTROCHEMISCHER SENSOR ZUR BESTIMMUNG DER BLUTGERINNUNG, EIN ENTSPRECHENDES BLUTGERINNUNGSMESSSYSTEM SOWIE EIN VERFAHREN ZUR BESTIMMUNG DER BLUTGERINNUNG**

ELECTROCHEMICAL SENSOR FOR DETERMINING BLOOD CLOTTING, CORRESPONDING SYSTEM FOR MEASURING BLOOD CLOTTING AND METHOD FOR DETERMINING BLOOD CLOTTING

CAPTEUR ELECTROCHIMIQUE PERMETTANT DE DETERMINER LA COAGULATION SANGUINE, SYSTEME CORRESPONDANT DE MESURE DE LA COAGULATION SANGUINE ET PROCEDE PERMETTANT DE DETERMINER LA COAGULATION SANGUINE

(84) Benannte Vertragsstaaten:
**AT BE CH CY DE DK ES FI FR GB GR IE IT LI LU MC NL PT SE TR**

(30) Priorität: **21.02.2000 DE 10007910**
**22.02.2000 US 184059 P**
**04.04.2000 DE 10016775**

(43) Veröffentlichungstag der Anmeldung:
**04.12.2002 Patentblatt 2002/49**

(73) Patentinhaber:
- **Roche Diagnostics GmbH**
  **68298 Mannheim (DE)**
  Benannte Vertragsstaaten:
  **DE**
- **F. Hoffmann-La Roche AG**
  **4070 Basel (CH)**
  Benannte Vertragsstaaten:
  **AT BE CH CY DK ES FI FR GB GR IE IT LI LU MC NL PT SE TR**

(72) Erfinder:
- **UNKRIG, Volker**
  **68526 Ladenburg (DE)**
- **MARQUANT, Michael**
  **68309 Mannheim (DE)**
- **HINDELANG, Fritz**
  **67316 Carlsberg (DE)**
- **KOTZAN, Holger**
  **68526 Ladenburg (DE)**
- **HORN, Carina**
  **64665 Alsbach-Haehnlein (DE)**
- **NORTMEYER, Christine**
  **68305 Mannheim (DE)**

(74) Vertreter: **Jung, Michael, Dr.**
**Roche Diagnostics GmbH**
**Patentabteilung**
**68298 Mannheim (DE)**

(56) Entgegenhaltungen:
**EP-A- 1 031 830**
**WO-A-98/39643**
**US-A- 4 304 853**

- **ARWIN, HANS ET AL: "Thrombin activity measured with a new electric method" FEBS LETT. (1976), 64(1), 95-7 , XP000995397**
- **RANDRIAMABAZAKA, H. N. ET AL: "Voltammetric assay of thrombin adsorbed on the carbon paste electrode." ELECTROANALYSIS, (1993) VOL. 5, NO. 3, PP. 231-241. , XP000995661**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **DE PEURIOT, M. DARAIO ET AL: "The electrochemical activity determination of trypsin-like enzymes. V. A comparative study of the electrochemical oxidation of the marker, p-aminodiphenylamine, and of electrogenic substrates in human plasma, whole blood, and aqueous solutions" J. ELECTROCHEM. SOC. (1981), 128(6), 1233-8 , XP000995360**

## Beschreibung

Die Erfindung betrifft einen Sensor auf trockenchemischer Basis zur Bestimmung der Blutgerinnung, der auf einem inerten Träger mindestens 2 Elektroden aufweist, sowie ein trockenes Reagenz. Außerdem betrifft die Erfindung ein Blutgerinnungsmeßsystem enthaltend einen elektrochemischen Sensor und ein Strommeßgerät. Schließlich betrifft die Erfindung auch ein Verfahren zur Bestimmung der Blutgerinnung mittels eines elektrochemischen Sensors.

In EP-B 0 441 222 werden ein Verfahren und Sensorelektrodensystem zur elektrochemischen Bestimmung eines Analyts oder einer Oxidoreductase beschrieben. Die Patentschrift offenbart die Rolle einer reduzierbaren Substanz als Elektronenüberträger bei der Redoxreaktion eines zu bestimmenden Analyts auf eine Elektrode. Ein typischer Analyt ist Glucose, Lactat oder Redoxenzym, wie beispielsweise Glucose-Dehydrogenase oder Lactat-Dehydrogenase.

Ein elektrochemisches Verfahren zur Bestimmung von Proteasen und Antiproteasen ist aus EP-B 0 018 002 bekannt. Es wird dort ein Proteasen- oder Antiproteasensubstrat aus einem Oligopeptid, an das ein aromatisches oder heterocyclisches Amin oder Polyamin gebunden ist, eingesetzt. Das zu bestimmende Enzym spaltet die Bindung zwischen der carboxyterminalen Aminosäure und dem Amin oder Polyamin und die Menge des freigesetzten Amins oder Polyamins wird elektrochemisch bestimmt. Hier wird die Bestimmung von frei beweglichen Bestandteilen der Blutgerinnungskaskade in Lösung beschrieben.

Aus C. Bisson et al., "A microanalytical device for the assessment of coagulation parameters in whole blood", Technical Digest Solid-State Sensor and Actuator Workshop, Hilton Head Island, South Carolina, USA, June 8-11, 1998, ist ein elektrochemischer Sensor für die Gerinnungsdetektion bekannt. Diese Publikation beschreibt generell die Möglichkeit, elektrochemische Gerinnungsssensoren mit amperometrischer Detektion einzusetzen. Details über die verwendeten Substrate werden beispielsweise nicht offenbart. Zudem sind die beschriebenen Elektroden und Testdevices aufwendig herzustellen und somit für einen Massenmarkt unattraktiv.

Generell bleibt festzuhalten, dass sich im Stand der Technik jedoch kein einfacher und zuverlässiger Ansatz für elektrochemische Gerinnungssensoren und entsprechende Verfahren zur Gerinnungsdetektion findet.

Die vorliegende Erfindung stellt zur Lösung dieses Problems einen elektrochemischen Sensor, ein Blutgerinnungsmeßsystem und ein Verfahren zur Bestimmung der Blutgerinnung zur Verfügung, wie sie in den unabhängigen Patentansprüchen beschrieben sind. Bevorzugte Ausführungsformen sind in den abhängigen Ansprüchen angegeben.

Der erfindungsgemäße Sensor ist ein Analysenelement auf trockenchemischer Basis. Der Sensor enthält mindestens zwei Elektroden, von denen mindestens eine Elektrode eine sogenannte Arbeitselektrode ist. Er enthält in einer bevorzugten Ausführungsform keine klassische Referenzelektrode, wie beispielsweise eine Ag/AgCl-Referenzelektrode, sondern lediglich mindestens eine Arbeits- und eine Gegenelektrode. Die Elektroden können aus allen gängigen Elektrodenmaterialien, beispielsweise Metallen, Edelmetallen, Legierungen oder Graphit aufgebaut sein, vorzugsweise aus Edelmetallen, wie Gold oder Palladium, oder Graphit. Die unterschiedlichen Elektroden des Sensors können aus gleichen oder unterschiedlichen Materialien bestehen. In einer besonders bevorzugten Ausführungsform enthält der Sensor eine Arbeitselektrode und eine Gegenelektrode, die beide aus Palladium bestehen.

Das trockene Reagenz des erfindungsgemäßen Sensors enthält beispielsweise die Verbindung (I)

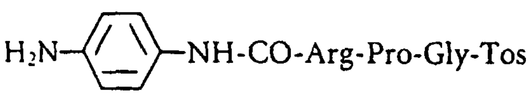

(I)

als Thrombinsubstrat. Dabei bedeutet Arg Arginin, Pro Prolin und Gly Glycin; Tos entspricht der Aminoschutzgruppe Tosyl. Generell sind als Peptidreste des erfindungsgemäßen Thrombinsubstrats alle Reste möglich, die von Thrombin abgespalten werden können, so daß ein elektrochemisch bestimmbares Phenylendiamin aus dem Substrat freigesetzt wird.

Das Enzym Thrombin ist die letzte Protease der Gerinnungskaskade und wird erst im Verlauf der Blutgerinnung aus dem Protein Prothrombin gebildet. Deshalb ist es möglich, die Verklumpung des Blutes durch Messung der Enzymaktivität des Thrombins zu verfolgen und so die Gerinnungszeit zu bestimmen.

Bei der Spaltung des Thrombinsubstrats entsteht ein p-Aminoanilin (Phenylendiamin), das an der Arbeitselektrode des Sensors oxidiert wird, wie es in EP-B 0 441 222 beschrieben ist. Die frei werdenden Elektronen werden detektiert. Grundsätzlich sind als elektrochemisch detektierbarer Teil des erfindungsgemäßen Thrombinsubstrats alle die Verbindungen einsetzbar, die als Elektronenüberträger (oder Mediator) 2. Art in EP-B 0 441 222 beschrieben sind.

Besonders vorteilhaft hat es sich bei der vorliegenden Erfindung herausgestellt als Verstärkersystem dem Reagenz Glucose-dye-oxidoreductase (GlucDOR) zuzusetzen, welches im zu untersuchenden Blut vorkommende Glucose oxidiert und hierzu den durch die Thrombinaktivität freigesetzten Elektronenüberträger 2. Art als Elektronenakzeptor benutzt.

Die Reaktionen finden nach folgendem Prinzip statt:

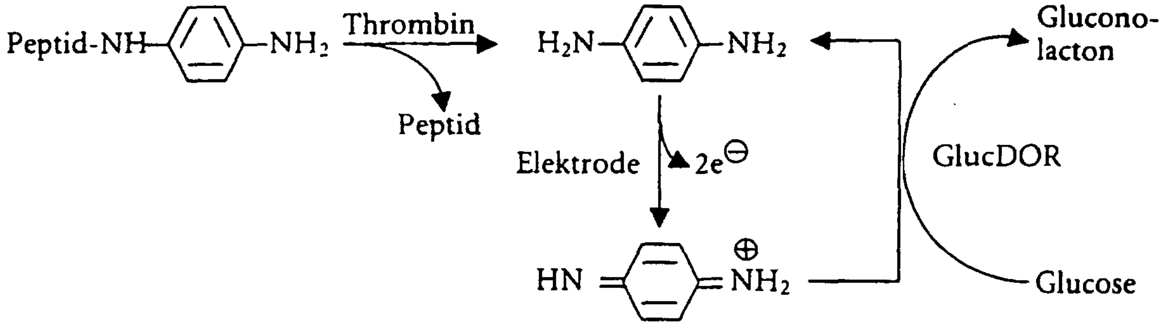

Das primäre Oxidationsprodukt des Elektronenüberträgers 2. Art ist ein Chinodiimin, welches mittels einer Dye-Oxidoreductase, wie beispielsweise Glucose-Dye-Oxidoreductase (GlucDOR) in p-Aminoanilin rückgeführt werden kann und damit erneut Elektronen an der Arbeitselektrode abgeben kann. Somit ist eine beträchtliche Verstärkung des ursprünglichen Signals möglich. Neben GlucDOR gibt es weitere Enzyme (wie z. B. Alkoholdehydrogenase oder Oxidasen wie Glucoseoxidase oder Laktatoxidase), die das Chinodiimin zum Phenylendiamin umsetzen können. Hierfür sind spezielle weitere Substrate (entsprechend der Glucose für GlucDOR) notwendig (wie z. B. Alkohole für das Enzym Alkoholdehydrogenase oder Laktat für Laktatoxidase), die dann gegebenenfalls in geeigneten Mengen in die Reagenzrezeptur eingebracht werden.

Die elektrochemische Bestimmung der Blutgerinnung erfolgt vorzugsweise quasi-potentiostatisch, bevorzugt mit einem 2-Elektrodensystem, bei dem die eine Elektrode gleichzeitig als Referenz- und Gegenelektrode, die andere Elektrode als Arbeitselektrode geschaltet ist. An dieses 2- Elektrodensystem wird eine konstante Spannung angelegt und der Strom über die Zeit gemessen. Dieses Verfahren wird auch als amperometrisches Meßverfahren bezeichnet. Für die Bestimmung der Blutgerinnungszeit wird der zeitliche Stromverlauf erfasst und ermittelt, nach welcher Zeitspanne ab dem Start der Gerinnungsmessung der gemessene Strom einen vorbestimmten Schwellenwert überschreitet. Diese Zeitspanne ist ein Maß für die Gerinnungszeit.

Neben dem beschriebenen amperometrischen Meßverfahren können auch voltametrische Meßverfahren eingesetzt werden. Hierbei wird keine konstante Spannung zwischen den Elektroden geregelt, sondern die Spannung wird linear von einem Startwert zu einem Endwert verändert und anschließend wieder zum Startwert zurück gefahren. Dieser Vorgang kann mehrfach über die gesamte Meßdauer wiederholt werden.

Beim voltametrischen Verfahren wird der Strom gegen die Spannung aufgetragen und man erhält dann entsprechend den Wiederholungen ineinandergeschachtelte Strom-Spannungskurven (Zyklovoltamogramme). Bei geeignetem Spannungsbereich bilden sich Oxidationspeaks und Reduktionspeaks des Elektronenüberträgers in diesen Kurven ab. Die Höhe dieser Peaks ist direkt proportional der Konzentration des Elektronenüberträgers, sofern nicht weitere redox-aktive Substanzen im überdeckten Potentialbereich mit oxidiert oder reduziert werden und damit einen zusätzlichen Strombeitrag liefern. Bei der Messung einer Konzentrationsänderung kann eine solche Störung gegebenenfalls vernachlässigt werden.

Trägt man nun die Stromwerte der Peak-Maxima oder die durch die Kurven eingeschlossenen Flächen (die dem Ladungsumsatz entsprechen) der einzelnen Strom-Spannungsschleifen über die Zeit auf, erhält man ebenfalls ein Bild der Konzentrationsänderung des Elektronenüberträgers über die Meßdauer in einem durch die Zyklusdauer gegebenen Zeitraster. Diese Information kann dann - wie bei den amperometrischen Verfahren - zur Bestimmung der Blutgerinnungszeit herangezogen werden.

Für einen Gerinnungstest, der auf die Bestimmung der Enzymaktivität einer Protease, z. B. Thrombin, zurückzuführen ist, werden Oligopeptid-Derivate der folgenden allgemeinen Formel (II) eingesetzt:

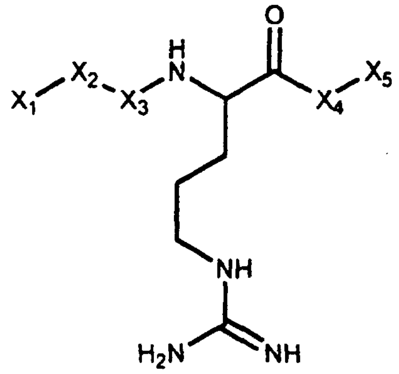

(II)

Dabei stellen $X_1$,$X_2$ und $X_3$ natürliche oder künstliche Aminosäuren inklusive eventueller Schutzgruppen dar. Die Sequenz $X_1$-$X_2$-$X_3$ wird je nach gewünschter Anwendung ausgewählt, beispielsweise für die Erkennung durch Thrombin, und ist für unterschiedliche Zwecke in zahlreichen Veröffentlichungen beschrieben worden. (vgl. z. B. U. Becker et al., Clin. Chem., **30** (4), 524-528 (*1984*); M.-C. Bouton et al., Eur. J. Biochem., **229** (2), 526-532 *( 1995);* H. D. Bruhn et al., Hämostaseologie, **15** (1), 54-56 (*1995*); E. De Candia et al., Thromb. Haemostasis, **77** (4), 735-740 (*1997*); P.L. Coleman et al., Clin. Chem., **29** (4), 603-608 ( 1983); J. DiMaio et al., J. Med. Chem., 35,3331-3341 *( 1992);* A. Frigola et al., J. Clin. Pathol., **32** (1), 21-25 (*1979*); P. J. Gaffney et al., Thromb. Res., **10** (4), 549-556 (*1977*); J. Hofsteenge et al., Biochem. J., 237, 243-251 (*1986*); R. Lottenberg et al., Thromb. Res., **28**, 313-332 *( 1982);* M.K. Ramjee, Anal. Biochem., **277**, 11-18 (*2000*); J. J. Slon-Usakiewicz et al., Biochem. **36**, 13494-13502 (*1997*); S. A. Sonder et al., Clin. Chem., **32** (6), 934-937 (*1986*); C. Soria et al., Thromb. Res., **19** (3), 435-440 (*1980*); T. Steinmetzer et al., J. Med. Chem., **42**, 3109-3115 (1999); A. Tripodi et al., Clin. Chem., **30** (8), 1392-1395 (*1984*); J. I. Witting et al., Thromb. Res., **46** (4), 567-574 (*1987*); EP-B 0 018 002; US 5,108,890; US 5,190,862; EP-A 0 280 610; EP-B 0 144 744) Darüber hinaus werden diese Aminosäuresequenzen teilweise auch von verschiedenen Firmen zur Erkennung unterschiedlicher Gerinnungsfaktoren kommerziell angeboten, beispielsweise von Pentapharm LTD, Basel, Schweiz oder von Chromogenix Germany, Haemochrom Diagnostica GmbH, Essen, Deutschland.

An die Aminosäuresequenz $X_1$-$X_2$-$X_3$ ist ein Molekülteil $X_4$-$X_5$ als Abgangsgruppe (leaving group) geknüpft, welcher durch die proteolytische Aktivität des Gerinnungsfaktors, beispielsweise also Thrombin, abgespalten wird. Der Molekülteil $X_4$-$X_5$, der zur Detektion der proteolytischen Aktivität genutzt wird, ist je nach gewünschtem Detektionsprinzip farbig (für die photometrische Bestimmung), fluoreszierend (siehe hierzu unter anderem M. K. Ramjee, Anal. Biochem., 277, 11-18 (2000)) oder elektroaktiv (vgl. EP-B 0 018 002).

Der Molekülteil $X_4$-$X_5$ ist im Rahmen der vorliegenden Erfindung elektroaktiv, das heißt an einer Elektrode unter Elektronenabgabe oder -aufnahme umsetzbar. Vorzugsweise weist $X_4$-$X_5$ ein Oxidationspotential von maximal 350mV gegen eine Ag/AgCl-Elektrode auf und kann durch eine elektrochemische Messung detektiert werden.

Dabei kann $X_4$ als Verknüpfungsatom bzw. -gruppe zur Oligopeptidsequenz N oder NH sein.

Der Molekülteil $X_5$ kann dabei folgende Struktur (III) haben,

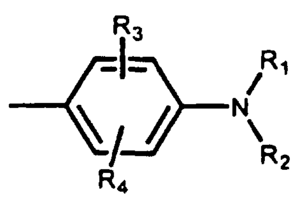

(III)

wobei $R_1$ und $R_2$ unabhängig voneinander Alkyl, Hydroxyalkyl, Alkoxyalkyl, Phosphoramidalkyl, Polyhydroxyalkyl, Sulfonalkyl, oder Wasserstoff sind, und $R_3$ und $R_4$ OH, O-Alkyl, Alkyl, H, Halogen, $NR_1R_2$, $CO_2R$, $CONR_1R_2$, $SR_1$, $NR_1$-CO-$R_1$, O-CO-$R_1$, H sind.

Peptidsubstrate dieses Typs für $X_4$ = N oder NH sind beispielsweise in den folgenden Patenten bzw. Patentanmeldungen beschrieben: JP-A 56042597; JP-A 58090535; WO-A 86/01209, US 5,059,525; JP-A 61112096; JP-A 03157353.

Alternativ kann $X_5$ folgende Struktur (IV) aufweisen:

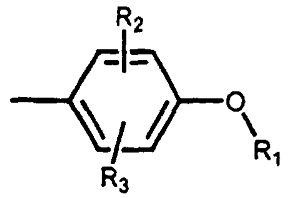

(IV)

in der $R_1$ bis $R_3$ die oben für die Formel III angegebene Bedeutung haben.

Peptidsubstrate dieses Typs sind für $X_4$ = NR bekannt (vgl. hierzu beispielsweise JP-A 03271299; EP-B 0 350 915; P. Kurzhals et al., Acta Pharm. Nord., 1 (5), 269-78 (1989); US 4,797,472; EP-B 0 224 254; WO-A 87/05608; EP-B 0 170 797; EP-B 0 167 980; EP-B 0 152 872; EP-B 0 076 042; JP-A 52148032).

Alternativ kann $X_5$ folgende Struktur (V) aufweisen:

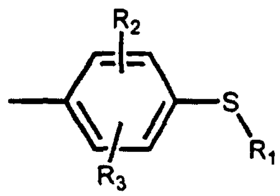

(V)

in der $R_1$ bis $R_3$ die oben zu Formel III und IV angegebene Bedeutung haben.

Peptidsubstrate dieses Typs mit $X_4$ = NR, O sind bekannt (vgl. hierzu beispielsweise T. Morimoto et al., Pept. Chem., Vol. Date 1989, 27th., 387-390 (1990); N. Nishino et al., Pept. Chem., Vol. Date 1988, 26th, 21-24 (1989); B. J. Johnson e tal., J. Org. Chem., 35 (1),255- 257 (1970)).

In einer bevorzugten Ausführungsform ist das Peptidsubstrat ein Thrombinsubstrat der allgemeinen Formel VI

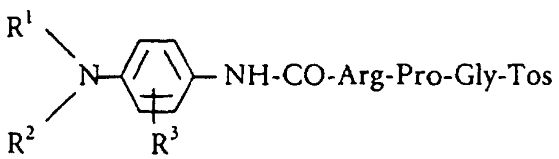

(VI)

wobei $R^1$ einen Alkylrest oder Wasserstoff,
$R^2$ einen Hydroxyalkylrest oder Wasserstoff und
$R^3$ Wasserstoff, Halogen, Alkoxy oder Alkylthio bedeutet.

Insgesamt kommen als Proteasesubstrate Verbindungen in Frage, die aus einem Peptidrest bestehen, der von einer Protease des Blutgerinnungssystems abgespalten werden kann und über das Carboxylende amidisch an substituierte Aniline, insbesondere an einen Phenylendiaminrest, gebunden ist.

Neben sogenannten Globaltests, bei denen die Gerinnungszeit als solche bestimmt wird und die zu diesem Zweck die Aktivität der Protease Thrombin bestimmen, können mit entsprechenden, dem Fachmann bekannten alternativen Substraten andere Gerinnungstests, z. B. ein anti-Faktor Xa-Test, oder die Bestimmung von einzelnen Gerinnungsfaktoren realisiert werden. Hierfür ist es notwendig, den Peptidteil des Substrats an die zu bestimmende Protease anzupassen. Für die erfindungsgemäße elektrochemische Bestimmung der Gerinnung oder einzelner Gerinnungsfaktoren ist es wichtig, dass ein elektrochemisch bestimmbares Abspaltprodukt des Peptidsubstrats gebildet und nachgewisen wird.

Zu den Globaltests zählen insbesondere die folgenden Tests: PT (Prothrombinzeit-Test), aPTT (aktivierte partielle Prothrombinzeit-Test) und ACT ("activated clotting time"-Test).

Die Erfindung wird durch die nachfolgenden Beispiele näher erläutert. Sämtliche Beispiele wurden für Vollblute als Probenmaterialien beschrieben. Die erfindungsgemäßen Sensoren und Verfahren sind jedoch auch für Citrat-Plasmen geeignet, wenn der Rezeptur des Testträgers oder der Probe Calcium zugesetzt wird. Weiterhin ist es erfindungsgemäß möglich, neben der in den Beispielen genannten Methode der Aufbringung des Reagenzes auf die Elektroden (Aktivatoren und Substrat werden gemeinsam auf die Elektroden dosiert und anschließend getrocknet) das Reagenz nicht direkt auf die Elektroden, sondern in der Nähe der Elektroden, beispielsweise neben den Elektroden

auf einem flachen Substrat, aufzubringen und zu trocknen. In diesem Fall wird das Reagenz mit der Blutprobe während der Messung zu den Elektroden transportiert. Alternativ dazu ist es möglich, das Reagenz in porösen Materialien wie zum Beispiel Vliesen, Papieren, Membranen und dergleichen unterzubringen, beispielsweise ablösbar zu imprägnieren. Diese Materialien müssen dabei von der Blutoder Plasmaprobe vor dem Kontakt mit den Elektroden durchströmt werden und dabei das Reagenz aufnehmen. Es ist auch möglich, einzelne Bestandteile des Reagenzes auf unterschiedliche Kompartimente des Testträgers aufzubringen, beispielsweise das Thrombinsubstrat auf oder direkt neben die Elektrode, den Aktivator aber räumlich getrennt davon (z.B. weiter entfernt von der Elektrode), oder beide Substanzen in unterschiedliche Schichten (z. B. Membranen oder Vliese) zu imprägnieren.

**Vergleichsbeispiel**

**Fertigung eines Gerinnungssensors für einen PT-Test**

**I. Beschreibung der elektrochemischen Funktion**

a) Offenes Zwei-Elektroden-System für "top-dosing" Probenaufgabe. Ebener Aufbau mit 2 Pd-Elektroden. Auf eine Pd-Elektrode wird eine Ag/AgCl-Paste (Typ: Acheson Electrodag 6037 SS) vollflächig dispensiert, so daß die gesamte Fläche dieser Pd-Elektrode von Ag/AgCl bedeckt ist.

Der elektrochemische Betrieb erfolgt quasi-potentiostatisch als 2-Elektroden-System, bei dem die Ag/AgCl Elektrode gleichzeitig als Referenz- und Gegenelektrode, die Pd-Elektrode als Arbeitselektrode geschaltet ist.

An dieses Zwei-Elektroden-System wird eine konstante Spannung angelegt und der Strom über der Zeit gemessen.

b) Ersatz der Silber-Silberchlorid-Referenzelektrode durch eine lösliche an der Gegenelektrode reduzierbare Substanz ("Mediator 2. Art"):

Es wird eine lösliche reduzierbare Substanz eingesetzt, die an der Gegenelektrode Elektronen aufnimmt und damit den Stromtransport durch die Gegenelektrode gewährleistet. Gleichzeitig wird durch das Reduktionspotential dieser Substanz und durch die an den Sensor zwischen der Arbeits- und der Gegenelektrode angelegte konstante Spannung das an der Arbeitselektrode erreichbare Oxidationspotential begrenzt. Das an der Arbeitselektrode durch Oxidation nachzuweisende p-Aminoanilin muß innerhalb des erreichbaren Oxidationspotentials liegen. Das Potential an der Arbeitselektrode gegen eine hypothetische Silber-Silberchlorid-Bezugselektrode muß sich im beschriebenen Zweielektrodensystem so einstellen, daß der Strom durch die Arbeitselektrode (hier Anode) und der Strom durch die Gegenelektrode (hier Kathode) dem Betrag nach gleich sind. Die eingesetzte reduzierbare Substanz darf nicht gleichzeitig an der Arbeitselektrode innerhalb des maximal erreichbaren Oxidationspotentials oxidierbar sein, da sonst das Signal des durch die vorgelagerte enzymatische Reaktion abgespaltene p-Aminoanilin überlagert wird.

**II. Herstellung eines trockenchemischen Sensors für einen PT Test**

a) Rezeptur der Reagenzmasse:

| Rezeptur 1: Ag/AgCl- Elektrodenausführung wie unter 1.I.a) beschrieben: | | | |
|---|---|---|---|
| Konzentration (in Klammern: erfindungsgemäß möglicher Bereich) | Substanz | Funktion der Substanz | Lieferant |
| 0,6% (0,1-5%) | Avicel RC 591 (carboxylierte mikrokristalline Zellulose) | Filmbildner | FMC-Corporation |
| 2% (0 - 5%) | Natrosol 250 M | Verdicker | Aqualon |
| 0,05% (0-5%) | Polyox 750 | Filmbildner | Union Carbide |

(fortgesetzt)

| Rezeptur 1: Ag/AgCl- Elektrodenausführung wie unter 1.I.a) beschrieben: | | | |
|---|---|---|---|
| Konzentration (in Klammern: erfindungsgemäß möglicher Bereich) | Substanz | Funktion der Substanz | Lieferant |
| 0,9% (0,05 - 5%) | Triton X 100 | Detergenz | Sigma |
| 200 mM (10 - 500 mM) | HEPES | Puffer | Roche |
| 0,1% (0,01-1%) | RSA ( Rinderserumalbumin) | Stützprotein | Roche |
| 0,1µg/ml (0,01-2µg/ml) | rhTF (human recombinant Tissue factor) | Aktivator des extrinsischen Weges der plasmatischen Gerinnung | Stago |

| Rezeptur 2: für den Einsatz des Mediators 2. Art gemäß 1.I.b) | | | |
|---|---|---|---|
| Konzentration (in Klammern: erfindungsgemäß möglicher Bereich) | Substanz | Funktion der Substanz | Lieferant |
| 0,6% (0,1-5%) | Avicel RC 591 (carboxylierte mikrokristalline Zellulose) | Filmbildner | FMC-Corporation |
| 2% (0 - 5%) | Natrosol 250 M | Verdicker | Aqualon |
| 0,05% (0-5%) | Polyox 750 | Filmbildner | Union Carbide |
| 0,9% (0,05 - 5%) | Triton X 100 | Detergenz | Sigma |
| 200 mM (10 - 500 mM) | HEPES | Puffer | Roche |
| 0,1% (0,01-1%) | RSA (Rinderserumalbumin) | Stützprotein | Roche |
| 0,1µg/ml (0,01-2µg/ml) | rhTF (human recombinant Tissue factor) | Aktivator des extrinsischen Weges der plasmatischen Gerinnung | Stago |
| 50 mM (1 - 250 mM) | p-Nitroso-Bis-Hydroxyethyl-anilin | Mediator 2. Art | Roche |

b) Dosierung und Trocknung

Von diesen Suspensionen werden jeweils5 µl auf die unter 1.I.a) und b) beschriebenen Sensoren dosiert und auf einem Bandtrockner oder im Trockenschrank bei 30 bis 50°C getrocknet. Rezeptur 1 eignet sich dabei für den Sensor gemäß 1.I.a); Rezeptur 2 eignet sich für den Sensor gemäß 1.I.b).

c) Bei Verwendung des so erhaltenen Sensors zur Bestimmung der Blutgerinnung wird eine Funktionskurve (Strom über Zeit), wie in Fig. 1 gezeigt, erhalten. Fig. 2 gibt die Funktionskurve eines Sensors mit Referenzelektrode wieder.

**Vergleichsbeispiel**

**Herstellung eines trockenchemischen Sensors für einen ACT Test:**

a) Rezeptur der Reagenzmasse

| Rezeptur (Alternative 1): | | | |
|---|---|---|---|
| Konzentration (in Klammern: erfindungsgemäß möglicher Bereich) | Substanz | Funktion der Substanz | Lieferant |
| 0,6% (0,1-5%) | Avicel RC 591 (carboxylierte mikrokristalline Zellulose) | Filmbildner | FMC-Corporation |
| 2% (0 - 5%) | Natrosol 250 M | Verdicker | Aqualon |
| 0,05% (0-5%) | Polyox 750 | Filmbildner | Union Carbide |
| 0,9% (0,05 - 5%) | Triton X 100 | Detergenz | Sigma |
| 200 mM (10 - 500 mM) | HEPES | Puffer | Roche |
| 0,1% (0,01-1%) | RSA (Rinderserumalbumin) | Stützprotein | Roche |
| 0,5% | Celite | Aktivator des intrinsischen Weges der plasmatischen Gerinnung | Sigma |

| Rezeptur (Alternative 2): | | | |
|---|---|---|---|
| Konzentration | Substanz | Funktion der Substanz | Lieferant |
| 30 mg/ml | Succrose | Stabilisator | Sigma |
| 10 mg/g | Gelatine | Filmbildner | American Gelatin |
| 0,1 mg/ml | Triton X 100 | Detergenz | Sigma |
| 40 mg/ml | Glycin | Puffer | Roche |
| 1% | RSA ( Rinderserumalbumin) | Stützprotein | Roche |
| 0,1μg/ml | rhTF (human recombinant Tissue factor) | Aktivator des extrinsischen Weges der plasmatischen Gerinnung | Stago |
| 3 mg/ml | Bovine Sulfatide | Aktivator des intrinsischen Weges der plasmatischen Gerinnung | Sigma |

b) Dosierung und Trocknung

Von jeweils einer dieser Suspensionen werden 5 μl auf dem unter 1. beschriebenen Sensor (Variante 1 a, d. h. mit Ag/AgCl-Referenzelektrode) dosiert und auf einem Bandtrockner oder im Trockenschrank bei 30 bis 50°C getrocknet.

**Erfindungsgemässes Beispiel**

**Amplifikation des Meßsignals:**

Aus dem Thrombinsubstrat wird durch Einwirkung der Gerinnungskaskade ein p-Amino-anilin (Phenylendiamin) freigesetzt. Dieses wird an der Arbeitselektrode oxidiert und die dabei freiwerdenden Elektroden detektiert. Das primäre Oxidationsprodukt ist dabei ein Chinodiimin. Durch enzymatische Rückführung dieses Oxidationsprodukts in das Phenylendiamin durch eine Dye-Oxidoreductase z.B. Glucose- Dye-Oxidoreductase (GlucDOR) ist die erneute Oxidation an der Elektrode und damit eine Verstärkung des ursprünglichen Signals möglich.

a) Rezeptur der Reagenzmasse:

| Konzentration (in Klammern: erfindungsgemäß möglicher Bereich) | Substanz | Funktion der Substanz | Lieferant |
|---|---|---|---|
| 0,6% (0,1-5%) | Avicel RC 591 (carboxylierte mikrokristalline Zellulose) | Filmbildner | FMC-Corporation |
| 2% (0 - 5%) | Natrosol 250 M | Verdicker | Aqualon |
| 0,05% (0-5%) | Polyox 750 | Filmbildner | Union Carbide |
| 0,9% (0,05 - 5%) | Triton X 100 | Detergenz | Sigma |
| 200 mM (10 - 500 mM) | H EPES | Puffer | Roche |
| 0,1% (0,01-1%) | RSA (Rinderserumalbumin) | Stützprotein | Roche Diagnostics |
| 0,1µg/ml (0,01-2µg/ml) | RhTF (human recombinant Tissue factor) | Aktivator des extrinsischen Weges der plasmatischen Gerinnung | Stago |
| 1,2 KU/g (0,01-10 KU/g) | Gucose-Dye-Oxidoreductase (GlucDOR) | Enzym | Roche |

Die für das "Amplifikationsenzym" GlucDOR als Substrat notwendige Glucose ist Bestandteil der Probe (ca. 10 mM) und wurde deshalb nicht in die Rezeptur eingearbeitet. Glucosezusatz ist aber prinzipiell möglich.

b) Dosierung und Trocknung

Von dieser Suspension werden 5 µl auf dem unter I.I.a. beschriebenen Sensor dosiert und auf einem Bandtrockner oder im Trockenschrank bei 30 bis 50°C getrocknet.

**Vergleichsbeispiel**

**Elektrochemischer ACT-Test**

a) Testaufbau

Der in diesem Beispiel verwendete Testaufbau (Sensor) entsprach dem in 1.I.b) beschriebenen Sensor (Verwendung eines "Mediators 2. Art").

b) Rezeptur der Reagenzmasse

| Konzentration (in Klammern: erfindungsgemäß möglicher Bereich) | Substanz | Funktion der Substanz | Lieferant |
|---|---|---|---|
| 0,6% (0,1-5%) | Avicel RC 591 (carboxylierte mikrokristalline Zellulose) | Filmbildner | FMC-Corporation |
| 2% (0 - 5%) | Natrosol 250 M | Verdicker | Aqualon |
| 0,05% (0-5%) | Polyox 750 | Filmbildner | Union Carbide |
| 0,9% (0,05 - 5% ) | Triton X 100 | Detergenz | Sigma |
| 200 mM (10 - 500 mM) | HEPES | Puffer | Roche |

(fortgesetzt)

| Konzentration (in Klammern: erfindungsgemäß möglicher Bereich) | Substanz | Funktion der Substanz | Lieferant |
|---|---|---|---|
| 0,1% (0,01-1%) | RSA (Rinderserumalbumin) | Stützprotein | Roche |
| 0,2 µg/ml (0,01 - 2µg/ml) | rhTF (human recombinant Tissue Factor) | Aktivator des extrinsischen Weges der plasmatischen Gerinnung | Stago |
| 6 mg/ml (0,5 - 50 mg/ml) | Bovine Sulfatide | Aktivator des intrinsischen Weges der plasmatischen Gerinnung | Life Science Reserach |
| 50 mM (1 - 250 mM) | p-Nitroso.Bis-Hydroxyethylanilin | Mediator 2. Art | Roche |

c) Dosierung und Trocknung

Von dieser Suspension wurden 5 µl auf den unter a) beschriebenen Sensor dosiert und auf einem Bandtrockner oder im Trockenschrank bei 30 bis 50 °C getrocknet.

d) Messergebnisse für den ACT-Test

Mit Vollblutproben, denen 1 bis 6 U Heparin je ml zugesetzt wurde ("Heparin-Spike"), wurde mit dem oben beschriebenen Sensor ein ACT-Test durchgeführt. Es wurde ein Potential von 300 mV angelegt und bei einem Schwellenwert von 0,5 µA die Zeit abgelesen. Die Ergebnisse sind in der folgenden Tabelle wiedergegeben:

| Heparin-Konzentation [U/ml] | Gerinnungszeit [s] |
|---|---|
| 1 | 47 |
| 2 | 100 |
| 3 | 172 |
| 4 | 235 |
| 5 | 279 |
| 6 | 353 |

**Vergleichsbeispiel**

**Elektrochemischer aPTT-Test**

a) Testaufbau:

Der in diesem Beispiel verwendete Testaufbau entsprach dem in Beispiel 1 unter I.I.b) beschriebenen Aufbau.

b) Rezeptur der Reagenzmasse:

| Konzentration (in Klammern: erfindungsgemäß möglicher Bereich) | Substanz | Funktion der Substanz | Lieferant |
|---|---|---|---|
| 0,6% (0,1-5%) | Avicel RC 591 (carboxylierte mikrokristalline Zellulose) | Filmbildner | FMC-Corporation |
| 2% (0 - 5%) | Natrosol 250 M | Verdicker | Aqualon |
| 0,05% (0-5%) | Polyox 750 | Filmbildner | Union Carbide |
| 0,9% (0,05 - 5%) | Triton X 100 | Detergenz | Sigma |
| 200 mM (10 - 500 mM) | HEPES | Puffer | Roche |
| 0,1% (0,01-1%) | RSA (Rinderserumalbumin) | Stützprotein | Roche |
| 0,6 mg/ml (0,05-10 mg/ml) | Soy bean phospatides | Aktivator des intrinsischen Weges der plasmatischen Gerinnung | Sigma |
| 1 mg/ml (0,1-10 mg/ml) | Bovine Sulfatide | Aktivator des intrinsischen Weges der plasmatischen Gerinnung | Life Science Research |
| 50 mM (1 - 250 mM) | p-Nitroso-Bis-Hydroxyethyl-anilin | Mediator 2. Art | Roche |

c) Dosierung und Trocknung

Von dieser Suspension werden 5 µl auf dem unter 1.I.b) beschriebenen Sensor dosiert und auf einem Bandtrockner oder im Trockenschrank bei 30 bis 50°C getrocknet.

d) Messergebnisse für den aPTT-Test

Mit Vollblutproben, denen 0 bis 0,35 U Heparin je ml zugesetzt wurde ("Heparin-Spike"), wurde mit dem oben beschriebenen Sensor ein aPTT-Test durchgeführt. Es wurde ein Potential von 300 mV angelegt und bei einem Schwellenwert von 0,5 µA die Zeit abgelesen. Die Ergebnisse sind in der folgenden Tabelle wiedergegeben:

| Heparin-Konzentation [U/ml] | Gerinnungszeit [s] |
|---|---|
| 0 | 59 |
| 0,15 | 79 |
| 0,25 | 117 |
| 0,35 | 164 |

**Vergleichsbeispiel**

**Kompensation des Hämatokriteffekts**

I. Beschreibung der Methode:

Bei chronoamperometrischen oder voltammetrischen Meßverfahren ist der aus einer Oxidation oder Reduktion resultierende Strom bei Verwendung von Vollblut als Analyten vom Hämatokritgehalt abhängig. Bei Auswertemethoden, die auf eine vorgegebene Stromschwelle zur Bestimmung der Gerinnungszeit basieren, bekommt man so abhängig vom Hämatokritgehalt unterschiedliche Gerinnungszeiten.

Der Hämatokritgehalt läßt sich durch einen speziellen Meßalgorithmus quasi messen und zur Korrektur der Stromwerte heranziehen. Dazu wird vor der eigentlichen chronoamperometrischen Messung eine kurze Messung des Scheinwiderstandes des mit Blut benetzten Sensors durchgeführt. Hierzu wird zwischen Arbeits- und Gegenelektrode

eine Wechselspannung mit einer Frequenz von 2 kHz und einer Amplitude von etwa 10 mV angelegt und der resultierende Effektivwert des durch den Sensor fließenden Wechselstroms gemessen. Durch Dividieren des Effektivwertes der angelegten Spannung mit dem Effektivwert des Wechselstroms erhält man den Scheinwiderstand Z des mit der Blutprobe befüllten Sensors. Dieser Scheinwiderstand Z ist abhängig von der Temperatur und dem Hämatokritgehalt des Blutes. Unter thermostatisierten isothermen Bedingungen läßt sich damit der Hämatokritgehalt im Blut unabhängig von der Konzentration des eigentlichen Elektronenüberträgers (Mediator) messen.

Mit folgender Formel lassen sich dann die Stromwerte der eigentlichen chronoamperometrischen Nachweismessung ($i_{mess}$) korrigieren um so vom Hämatokritgehalt unabhängige Ergebnisse ($i_{korr}$) zu erhalten :

$$i_{korr} = i_{mess} \text{ x } ((Z - Z_{median}) \text{ x } 0.001 + 1)$$

wobei Z der Scheinwiderstand der aktuellen Messung und $Z_{median}$ der Scheinwiderstand aus der Mitte vieler Blutproben ist und sich als Ergebnis einer Chargenkalibration ergibt.

II. Versuchsdurchführung

II.1 Herstellung des trockenchemischen Formats für den verwendeten PT-Test:

Die Kompensation des Hämatokriteffekts wurde am Beispiel eines PT-Tests gezeigt. Der in diesem Beispiel verwendete Testaufbau entsprach dem in Beispiel 1 unter Ib) beschriebenen Aufbau. Die verwendete Rezeptur war identisch mit der in Beispiel 1 unter II a) aufgeführten Rezeptur 2.

II.2. Einstellung der Hämatokritwerte

Frisches Venenblut wurde auf dem Eisbad auf 0°C gekühlt. Aliquots wurden in einer Tischzentrifuge in Erythrozten und Zellpellet getrennt. Höhere Hämatokrite wurden durch Entfernen eines Teils des Plasmas und Resuspension der Zellen in dem verbliebenen Plasma eingestellt. Niedrigere Hämatokrite wurden durch Zufügen von Plasma, das aus einen anderen Aliquot desselben Blutes wie beschrieben gewonnen worden war, eingestellt.

Die Versuchsergebnisse sind in der folgenden Tabelle wiedergegeben:

| Hämatokrit [%] | Gerinnungszeiten [s] | |
|---|---|---|
| | unkorrigiert | korrigiert |
| 35 | 39 | 52 |
| 45 | 45 | 53 |
| 65 | 78 | 56 |

**Vergleichsbeispiel**

**Voltammetrische Methode**

Neben dem beschriebenen amperometrischen Meßverfahren können auch voltametrische Meßverfahren eingesetzt werden. Es wird keine konstante Spannung zwischen den Elektroden geregelt, sondern die Spannung wird linear von einem Startwert zu einem Endwert verändert und anschließend wieder zum Startwert zurück gefahren. Dieser Vorgang wird mehrfach über die gesamte Meßdauer wiederholt. Bei diesem Verfahren wird dann der Strom gegen die Spannung aufgetragen und man erhält dann entsprechend den Wiederholungen in einander geschachtelte Strom-Spannungskurven (Zyklovoltamogramme). Bei geeignetem Spannungsbereich bilden sich Oxidationspeaks und Reduktionspeaks des Elektronenüberträgers in diesen Kurven ab.

In Fig. 2 ist ein Beispiel für solche aufeinanderfolgende Voltamogramme dargestellt. Es wurde ein Potentialbereich von 100 bis 600 mV und zurück über die gesamte Meßdauer wiederholt durchlaufen.

Die Höhe dieser Peaks ist direkt proportional der Konzentration des Elektronenüberträgers, sofern nicht weitere redox aktive Substanzen im überdeckten Potentialbereich mitoxidiert oder mitreduziert werden und damit einen zusätzlichen Strombeitrag liefern. Bei der Messung einer Konzentrationsänderung kann eine solche Störung gegebenenfalls vernachlässigt werden.

Trägt man nun die Stromwerte der Peak-Maxima oder die durch die Kurven eingeschlossenen Fläche (Ladungsumsatz) der einzelnen Strom-Spannungsschleifen über die Zeit auf, erhält man ebenfalls ein Bild der Konzentrationsänderung des Elektronenüberträgers über die Meßdauer in einem durch die Zyklusdauer gegebenen Zeitraster. In Fig. 3 ist der Ladungsinhalt (Summe der Stromwerte / Meßdichte) der aufeinanderfolgenden zyklischen Voltamogramme über die Zeit dargestellt. Deutlich ist der Verlaufsunterschied zwischen dem Normalen Gerinnungsbereich und dem Ergebnis bei einem Marcumar-Probanden (mit verlangsamter Blutgerinnung) zu erkennen.

Zur Bestimmung bzw. zum Ablesen einer "Gerinnungszeit" interessiert in erster Linie der Zeitpunkt, wann die Ladung eines zyklischen Voltamogramms anfängt, zu steigen und wie schnell dieses geschieht. Das bedeutet also, wann startet die Gerinnung (Thrombin ist aktiviert) und wie schnell läuft diese ab (wie schnell wird wieviel Thrombin zusätzlich aktiviert). Aus diesem Grund wird wie in Fig. 4 dargestellt, die zeitliche Ableitung aus den Kurven von Fig. 3, also dQ nach dt gebildet. Das Maximum der Steigung ist in der zeitlichen Ableitung das Maximum. Die Zeit t des Maximums kann als Gerinnungszeit abgelesen werden.

Für die in den Figuren 3 und 4 dargestellten Beispiele erhält man dann folgendes Ergebnis:

| | Zeit zum Maximum dQ/dt | |
|---|---|---|
| Messung | Normalbereich | Marcumar Proband |
| 1 | 74 | 208 |
| 2 | 70 | 200 |
| 3 | 72 | 196 |
| 4 | 74 | 200 |
| 5 | 72 | 196 |
| 6 | 74 | 188 |
| 7 | 74 | 208 |
| 8 | 74 | 216 |
| 9 | 74 | 204 |
| 10 | 74 | 234 |
| 11 | 76 | 218 |

## Patentansprüche

1. Elektrochemischer Sensor auf trockenchemischer Basis zur Bestimmung der Blutgerinnung oder einzelner Gerinnungsfaktoren, der auf einem inerten Träger mindestens zwei Elektroden aufweist, sowie ein trockenes Reagenz, wobei das Reagenz ein Proteasesubstrat enthält, das aus einem Peptidrest besteht, der von einer Protease des Blutgerinnungssystems abgespalten werden kann, und über das Carboxylende amidisch an einen Phenylendiaminrest gebunden ist, **dadurch gekennzeichnet, daß** das Reagenz auch eine Dye-Oxidoreductase enthält, die in der Lage ist, die Umsetzung eines Chinodiimins in ein Phenylendiamin zu katalysieren.

2. Elektrochemischer Sensor gemäß Anspruch 1, **dadurch gekennzeichnet, daß** er zwei Elektroden enthält, die aus dem gleichen Material bestehen.

3. Elektrochemischer Sensor gemäß Anspruch 2, **dadurch gekennzeichnet, daß** das Elektrodenmaterial Palladium oder Gold ist

4. Elektrochemischer Sensor gemäß einem der Ansprüche 1 bis 3, **dadurch gekennzeichnet, dass** die Protease Thrombin und das Proteasesubstrat ein Thrombinsubstrat ist.

5. Elektrochemischer Sensor gemäß Anspruch 4, **dadurch gekennzeichnet, daß** das Thrombinsubstrat eine Verbindung der Formel VI ist,

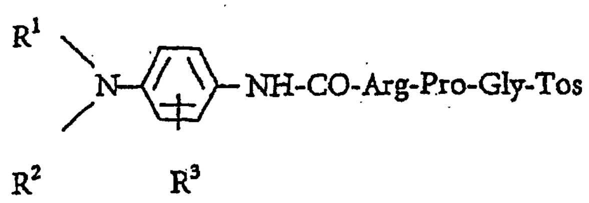

(VI)

wobei
R1 einen Alkylrest, einen Hydroxyalkylrest oder Wasserstoff,
R2 einen Hydroxyalkylrest oder Wasserstoff und
R3 Wasserstoff, Halogen, Alkoxy oder Alkylthio bedeutet.

6. Elektrochemischer Sensor gemäß einem der Ansprüche 1 - 5, **dadurch gekennzeichnet, daß** die Dye-Oxidoreductase Glucose-Dye-Oxidoreductase ist.

7. Blutgerinnungsmeßsystem enthaltend einen elektrochemischen Sensor und ein Strommeßgerät, **dadurch gekennzeichnet, daß** als Sensor ein Sensor gemäß einem der Ansprüche 1 - 6 verwendet wird.

8. Verfahren zur Bestimmung der Blutgerinnung mittels eines elektrochemischen Sensors, **dadurch gekennzeichnet, daß** an einen Sensor gemäß einem der Ansprüche 1 - 6 eine konstante Spannung angelegt, die zu untersuchende Blutprobe so auf den Sensor aufgegeben wird, daß das Reagenz und die Elektroden befeuchtet werden und der zwischen den Elektroden fließende Strom über die Zeit gemessen wird.

9. Verfahren gemäß Anspruch 8, **dadurch gekennzeichnet, daß** damit die Prothrombinzeit (PT), die aktivierte partielle Prothrombinzeit (aPTT) oder die "activated clotting time" (ACT) gemessen wird.

10. Verfahren gemäß Anspruch 8 oder 9, **dadurch gekennzeichnet, daß** bei der Bestimmung der Blutgerinnungszeit eine Korrektur des Hämatokriteinflusses über eine Impedanzmessung erfolgt.

11. Reagenz zur Bestimmung der Blutgerinnung enthaltend ein Proteasesubstrat, das aus einem Peptidrest besteht, der von einer Protease des Blutgerinnungssystems abgespalten werden kann und über das Carboxylende amidisch an einen Phenplendiaminrest gebunden ist, **dadurch gekennzeichnet, daß** es eine Dye-Oxidoreductase enthält.

12. Reagenz gemäß Anspruch 11, **dadurch gekennzeichnet, daß** die Dye-Oxidoreductase Glucose-Dye-Oxidoreductase ist.

13. Reagenz gemäß Anspruch 11 oder 12, **dadurch gekennzeichnet, daß** die Protease Thrombin und das Proteasesubstrat ein Tbrombinsubstrat ist.

14. Verfahren zur Bestimmung der Blutgerinnung mittels eines elektrochemischen Sensors, **dadurch gekennzeichnet, daß** an einen Sensor gemäß einem der Ansprüche 1 - 6 eine sich linear ändernde Spannung angelegt, die zu untersuchende Blutprobe so auf den Sensor aufgegeben wird, daß das Reagenz und die Elektroden befeuchtet werden und der zwischen den Elektroden fließende Strom über die Zeit gemessen wird.

15. Verfahren gemäß Anspruch 14, **dadurch gekennzeichnet, daß** bei der Bestimmung der Blutgerinnungszeit eine Korrektur des Hämatokriteinflusses über eine Impedanzmessung erfolgt.

## Claims

1. Electrochemical sensor based on dry chemistry for the determination of blood coagulation or individual coagulation factors which has at least two electrodes on an inert support and a dry reagent, wherein the reagent contains a protease substrate which is composed of a peptide group which can be cleaved by a protease of the blood coagulation system and is bound via its carboxyl end to a phenylenediamine residue by means of an amide linkage, **characterized in that** the reagent also contains a dye-oxidoreductase which is able to catalyse the conversion of a quinodiimine into a phenylenediamine.

**2.** Electrochemical sensor as claimed in claim 1, **characterized in that** it contains two electrodes which are composed of the same material.

**3.** Electrochemical sensor as claimed in claim 2, **characterized in that** electrode material is palladium or gold.

**4.** Electrochemical sensor as claimed in one of the claims 1 to 3, **characterized in that** the protease is thrombin and the protease substrate is a thrombin substrate.

**5.** Electrochemical sensor as claimed in claim 4, **characterized in that** the thrombin substrate is a compound of formula VI

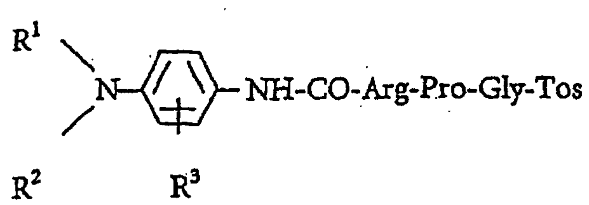

(VI)

in which
R1 denotes an alkyl residue, a hydroxyalkyl residue or hydrogen,
R2 denotes a hydroxyalkyl residue or hydrogen and
R3 denotes hydrogen, halogen, alkoxy or alkylthio.

**6.** Electrochemical sensor as claimed in one of the claims 1 - 5, **characterized in that** the dye-oxidoreductase is glucose-dye-oxidoreductase.

**7.** System for measuring blood coagulation comprising an electrochemical sensor and an instrument for measuring current, **characterized in that** a sensor as claimed in one of the claims 1 - 6 is used as the sensor.

**8.** Method for determining blood coagulation by means of an electrochemical sensor, **characterized in that** a constant voltage is applied to a sensor as claimed in one of the claims 1 - 6, the blood sample to be examined is applied to the sensor in such a manner that the reagent and the electrodes are moistened and the current flowing between the electrodes is measured over time.

**9.** Method as claimed in claim 8, **characterized in that** it is used to measure the prothrombin time (PT), activated partial prothrombin time (aPTT) or the activated clotting time (ACT).

**10.** Method as claimed in claim 8 or 9, **characterized in that** the determination of the blood clotting time is corrected for the haematocrit effect by means of an impedance measurement.

**11.** Reagent for the determination of blood coagulation containing a protease substrate which is composed of a peptide residue that can be cleaved by a protease of the blood coagulation system and is bound via its carboxyl end to a phenylenediamine residue by means of an amide linkage, **characterized in that** it contains a dye-oxidoreductase.

**12.** Reagent as claimed in claim 11, **characterized in that** the dye-oxidoreductase is a glucose-dye-oxidoreductase.

**13.** Reagent as claimed in claim 11 or 12, **characterized in that** the protease is thrombin and the protease substrate is a thrombin substrate.

**14.** Method for determining blood coagulation by means of an electrochemical sensor, **characterized in that** a voltage which changes linearly is applied to a sensor as claimed in one of the claims 1 - 6, the blood sample to be examined is applied to the sensor in such a manner that the reagent and the electrodes are moistened and the current flowing between the electrodes is measured over time.

**15.** Method as claimed in claim 14, **characterized in that** the determination of the blood clotting time is corrected for the haematocrit effect by means of an impedance measurement.

## Revendications

1. Capteur électrochimique basé sur la chimie sèche pour déterminer la coagulation sanguine ou des facteurs de coagulation individuels, qui présente au moins deux électrodes sur un support inerte, ainsi qu'un réactif sec, le réactif renfermant un substrat de protéase, qui est constitué d'un radical peptidique, qui peut être clivé par une protéase du système de coagulation, et qui est lié de façon amidique par la terminaison carboxyle à un radical phénylènediamine, **caractérisé en ce que** le réactif renferme également une colorant-oxydoréductase qui est en mesure de catalyser la conversion dune quinodiimine en une phénylènediamine.

2. Capteur électrochimique selon la revendication 1, **caractérisé en ce qu'**il renferme deux électrodes qui sont constituées de la même matière.

3. Capteur électrochimique selon la revendication 2, **caractérisé en ce que** le matériau des électrodes est du palladium ou de l'or.

4. Capteur électrochimique selon l'une quelconque des revendications 1 à 3, **caractérisé en ce que** la protéase est la thrombine et le substrat de protéase est un substrat de thrombine.

5. Capteur électrochimique selon la revendication 4, **caractérisé en ce que** le substrat de thrombine est un composé de formule VI,

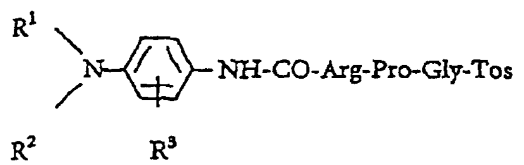 (VI)

dans laquelle
$R^1$ représente un radical alkyle, un radical hydroxyalkyle ou un atome d'hydrogène,
$R^2$ représente un radical hydroxyalkyle ou un atome d'hydrogène et $R^3$ représente un atome d'hydrogène, un atome d'halogène, un radical alcoxy ou alkylthio.

6. Capteur électrochimique selon l'une quelconque des revendications 1 à 5, **caractérisé en ce que** la colorant-oxydoréductase est la glucose colorant-oxydoréductase.

7. Système de mesure de la coagulation sanguine renfermant un capteur électrochimique et un ampèremètre, **caractérisé en ce qu'**un capteur selon l'une quelconque des revendications 1 à 6, est employé comme capteur.

8. Procédé pour déterminer la coagulation sanguine au moyen d'un capteur électrochimique, **caractérisé en ce qu'**une tension continue est appliquée à un capteur selon l'une quelconque des revendications 1 à 6, l'échantillon sanguin à analyser est déposé sur le capteur de sorte à humecter le réactif et les électrodes et le courant circulant entre les électrodes est mesuré au cours du temps.

9. Procédé selon la revendication 8, **caractérisé en ce que** le temps de prothrombine (PT), le temps de prothrombine partielle activée (aPTT) ou le temps de coagulation activée ("activated clotting time") (ACT) sont ainsi mesurés.

10. Procédé selon la revendication 8 ou 9, **caractérisé en ce que**, lors de la détermination du temps de coagulation sanguine, une correction de l'influence de l'hématocrite est réalisée par une mesure d'impédance.

11. Réactif pour déterminer la coagulation sanguine renfermant un substrat de protéase, qui est constitué d'un radical peptidique, qui peut être clivé par une protéase du système de coagulation, et qui est lié de façon amidique par la terminaison carboxyle à un radical phénylènediamine, **caractérisé en ce qu'**il renferme une colorant-oxydoréductase.

12. Réactif selon la revendication 11, **caractérisé en ce que** la colorant-oxydoréductase est la glucose colorant-oxydoréductase.

**13.** Réactif selon la revendication 11 ou 12, **caractérisé en ce que** la protéase est la thrombine et le substrat de protéase est un substrat de thrombine.

**14.** Procédé pour déterminer la coagulation sanguine au moyen d'un capteur électrochimique, **caractérisé en ce qu'**une tension à variation linéaire est appliquée à un capteur selon l'une quelconque des revendications 1 à 6, l'échantillon sanguin est déposé sur le capteur de sorte à humecter le réactif et les électrodes et le courant circulant entre les électrodes est mesuré au cours du temps.

**15.** Procédé selon la revendication 14, **caractérisé en ce que**, lors de la détermination du temps de coagulation sanguine, une correction de l'influence de l'hématocrite est réalisée par une mesure d'impédance.

Fig. 1

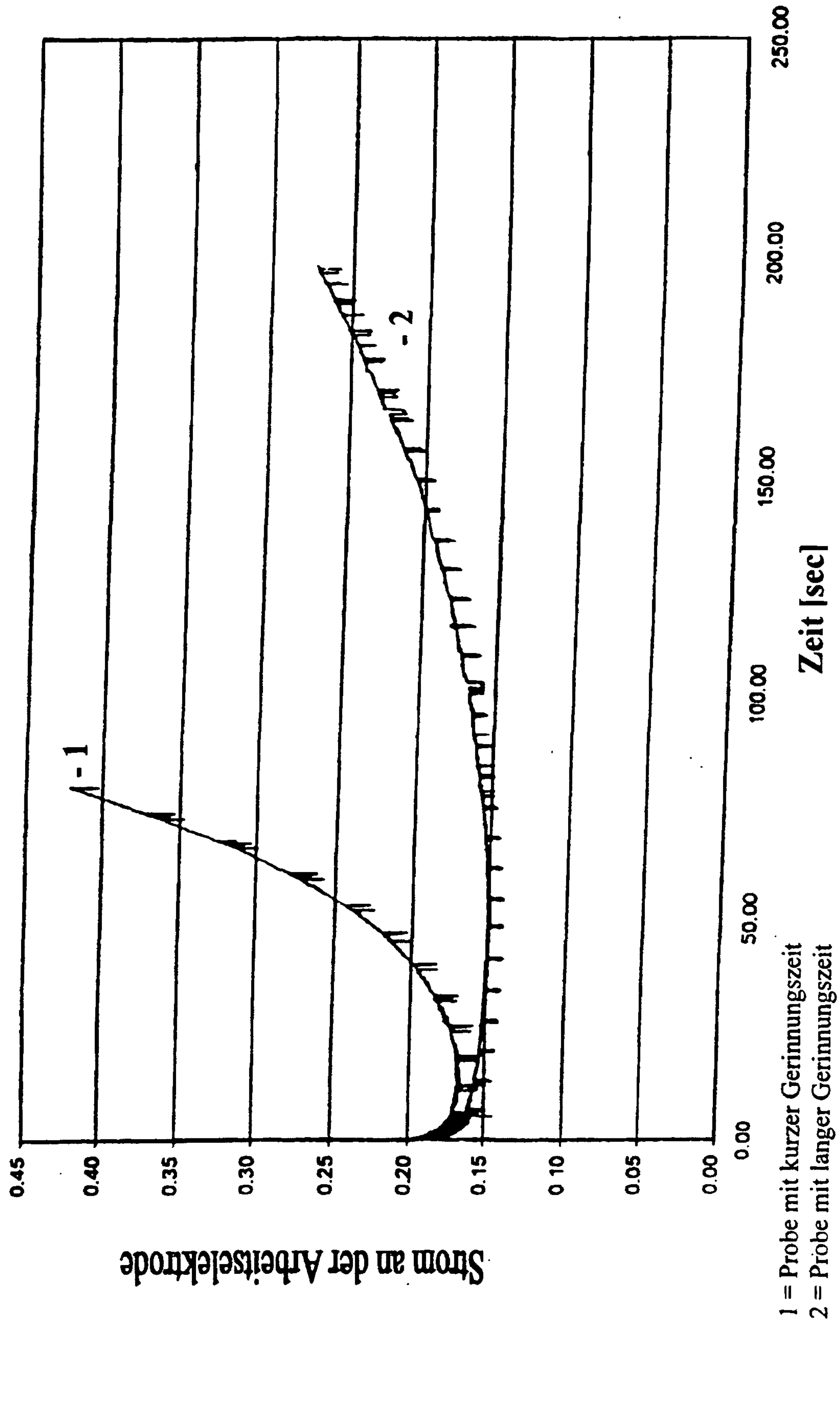
Strom an der Arbeitselektrode
0.45
0.40
0.35
0.30
0.25
0.20
0.15
0 10
0.05
0.00
0.00
50.00
100.00
150.00
200.00
250.00
Zeit [sec]
- 1
- 2
1 = Probe mit kurzer Gerinnungszeit
2 = Probe mit langer Gerinnungszeit

Fig. 2

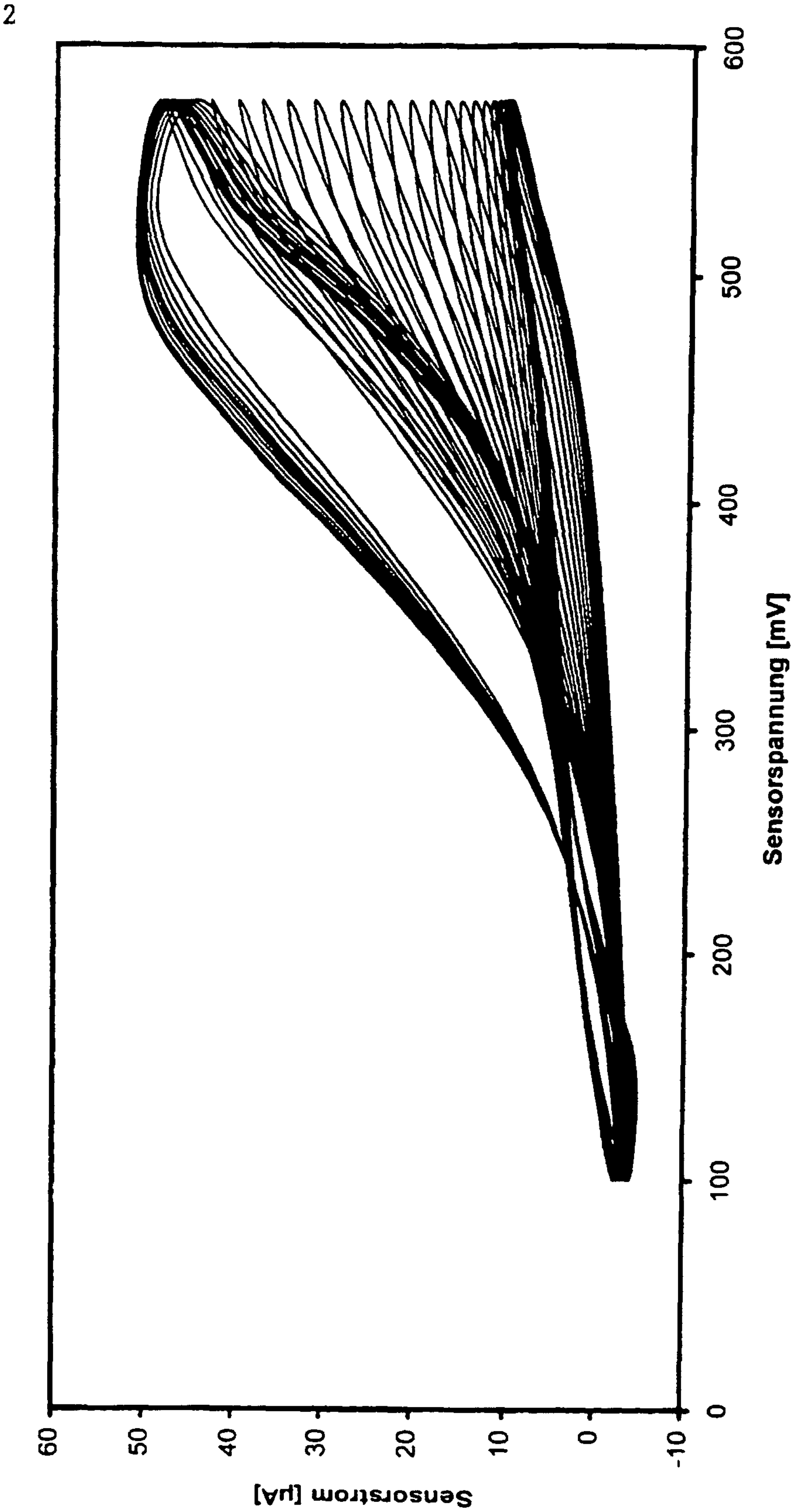
Sensorspannung [mV]
0
100
200
300
400
500
600
Sensorstrom [µA]
60
50
40
30
20
10
0
-10

Fig. 3

Fig. 3

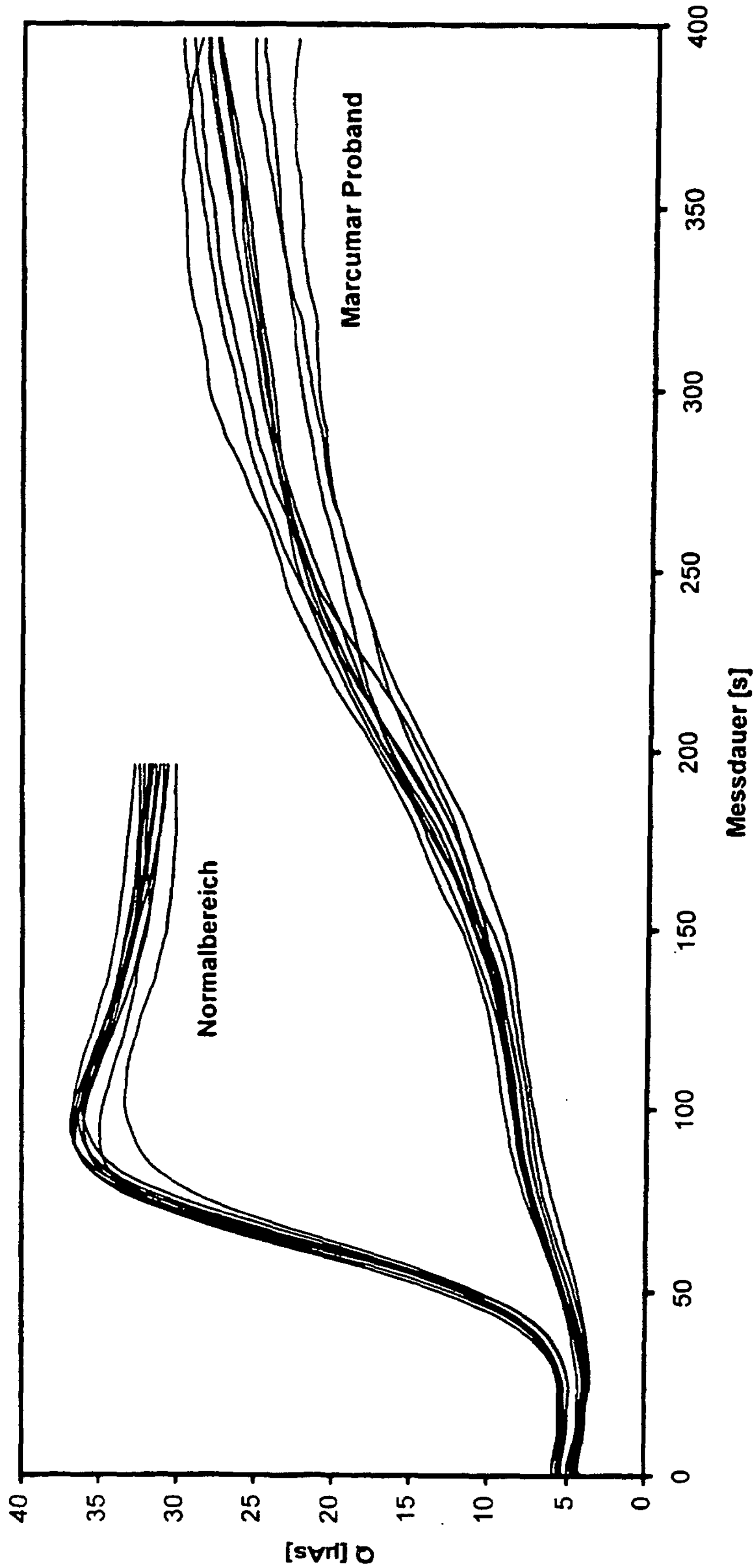
Normalbereich
Marcumar Proband
Messdauer [s]
Q [µAs]
0
50
100
150
200
250
300
350
400
0
5
10
15
20
25
30
35
40

Fig. 4

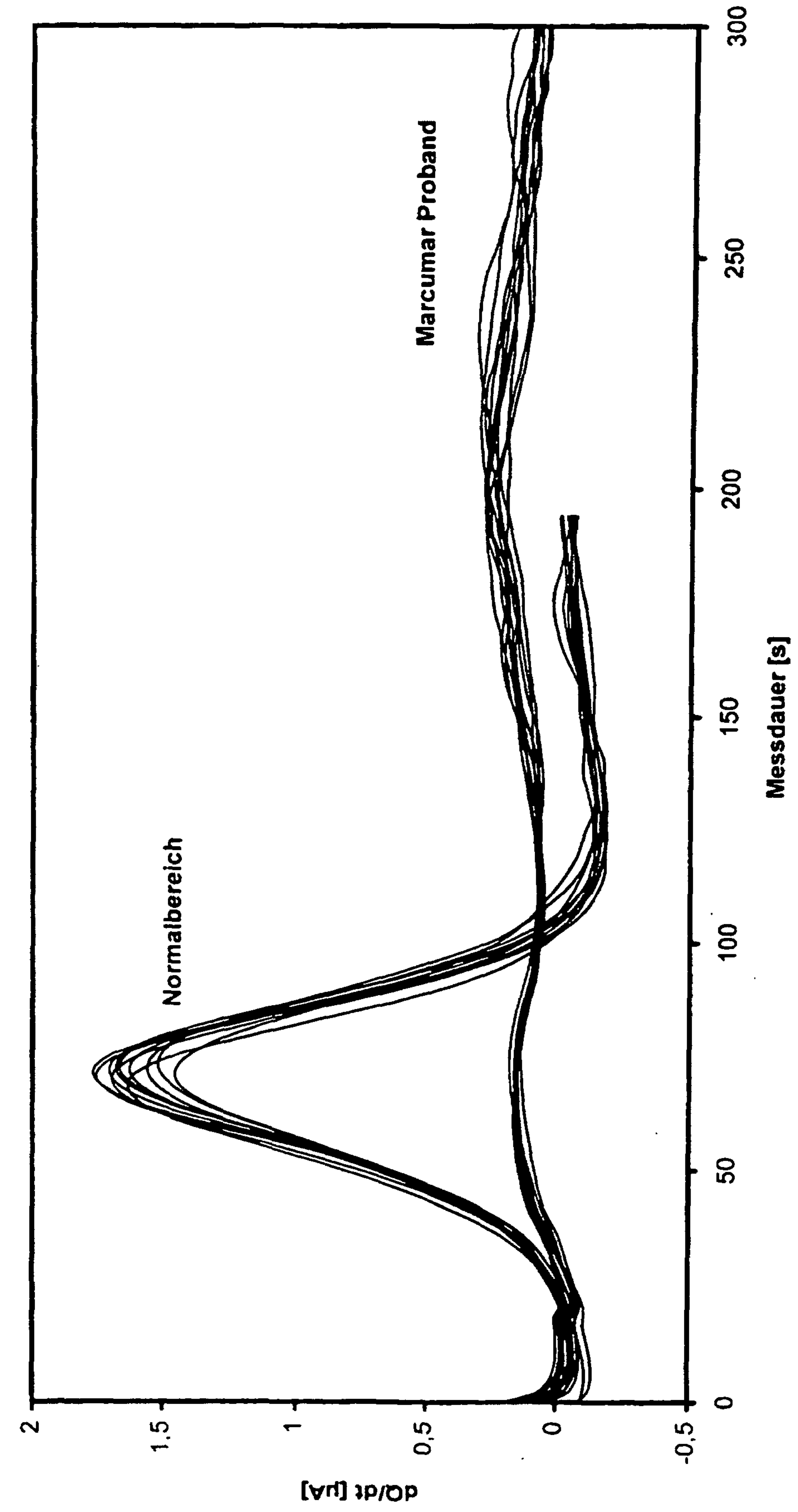
Normalbereich
Marcumar Proband
Messdauer [s]
dQ/dt [µA]
0
50
100
150
200
250
300
2
1,5
1
0,5
0
-0,5